(19) Europäisches Patentamt
European Patent Office
Office européen des brevets

(11) **EP 3 128 894 B1**

(12) **FASCICULE DE BREVET EUROPEEN**

(45) Date de publication et mention
de la délivrance du brevet:
**17.03.2021 Bulletin 2021/11**

(21) Numéro de dépôt: **15718544.8**

(22) Date de dépôt: **07.04.2015**

(51) Int Cl.:
*A61B 3/00* *(2006.01)*    *A61B 3/028* *(2006.01)*

(86) Numéro de dépôt international:
**PCT/FR2015/050892**

(87) Numéro de publication internationale:
**WO 2015/155458 (15.10.2015 Gazette 2015/41)**

(54) **RÉFRACTEUR ET PROCÉDÉ DE MESURE DE RÉFRACTION UTILISANT UN TEL RÉFRACTEUR**

PHOROPTER UND VERFAHREN ZUR MESSUNG DER REFRAKTION MITTELS EINES PHOROPTERS DES BESAGTEN TYPS

PHOROPTER, AND METHOD FOR MEASURING REFRACTION USING A PHOROPTOR OF SAID TYPE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.04.2014 FR 1453129**

(43) Date de publication de la demande:
**15.02.2017 Bulletin 2017/07**

(73) Titulaire: **Essilor International**
**94220 Charenton-le-Pont (FR)**

(72) Inventeurs:
• **BOUTINON, Stéphane**
  **F-94220 Charenton-le-Pont (FR)**
• **TEJEDOR DEL RIO, Vincent**
  **F-94220 Charenton-le-Pont (FR)**
• **NAUCHE, Michel**
  **F-94220 Charenton-le-Pont (FR)**

(74) Mandataire: **Jacobacci Coralis Harle**
**32, rue de l'Arcade**
**75008 Paris (FR)**

(56) Documents cités:
**WO-A1-2012/123549    US-A1- 2004 032 568
US-A1- 2006 106 426**

EP 3 128 894 B1

**Description**

DOMAINE TECHNIQUE AUQUEL SE RAPPORTE L'INVENTION

La présente invention concerne le domaine de l'optométrie.

Elle concerne plus particulièrement un réfracteur, ainsi qu'un procédé de mesure de réfraction utilisant un tel réfracteur.

ARRIERE-PLAN TECHNOLOGIQUE

Dans le cadre de la mesure de l'acuité visuelle d'un patient, il a déjà été proposé de simuler la compensation visuelle à fournir, par exemple au moyen de lunettes d'essai ou d'un réfracteur, tel qu'une tête de réfraction.

Les lunettes d'essais peuvent accueillir successivement des verres d'essai ayant des corrections différentes, jusqu'à trouver la correction qui convient au patient.

Cette solution n'est pas pratique et nécessite le stockage séparé des verres d'essai dans des boîtes dédiées. Elle implique en outre des changements de lentilles qui provoquent des transitions de puissance de correction non désirées et non continues.

Dans la tête de réfraction, les verres d'essai sont placés sur plusieurs disques, entraînés en rotation manuellement ou à l'aide d'un mécanisme motorisé.

On comprend toutefois qu'un tel objet présente un encombrement et un poids importants, liés au nombre de verres placés sur chaque disque. De ce fait, la tête de réfraction est nécessairement montée sur un support rigide, avec une direction d'observation substantiellement horizontale.

Un tel appareil n'est donc pas adapté pour établir précisément la correction requise pour le patient en vision de près. En effet, avec un tel appareil, l'axe visuel est maintenu quasiment horizontal même lorsque le praticien cherche à déterminer la correction requise en vision de près. Au contraire, en position naturelle, l'axe visuel est orienté de 30° vers le bas en vision de près, ce qui entraîne une amétropie légèrement différente de celle constatée avec l'axe visuel horizontal du fait du positionnement différent de l'œil et des muscles oculomoteurs.

Le document US 2004/0032568 divulgue un réfracteur selon le préambule de la revendication 1.

OBJET DE L'INVENTION

Dans ce contexte, la présente invention propose un réfracteur comprenant une enceinte comprenant une face avant présentant une première fenêtre optique et une face arrière présentant une seconde fenêtre optique alignée avec la première fenêtre optique selon un axe optique d'observation, et au moins un dispositif de compensation visuelle permettant d'observer selon l'axe optique d'observation, caractérisé en ce que le dispositif de compensation visuelle comprend, entre la première fenêtre et la seconde fenêtre, un premier élément optique de puissance sphérique selon l'axe optique variable, et en ce que l'enceinte est montée sur un support orientable mobile en rotation par rapport à une partie fixe autour d'un axe horizontal.

Le dispositif de compensation visuelle, qui permet notamment une variation de la puissance sphérique selon l'axe d'observation, est logé entre la première fenêtre et la seconde fenêtre formées dans l'enceinte, qui peut quant à elle être déplacée en rotation autour de l'axe horizontal.

On obtient ainsi un ensemble compact orientable de manière à effectuer les mesures d'acuité visuelle dans une position naturelle pour le patient, en particulier en vision de loin, en vision intermédiaire et en vision de près.

Le dispositif de compensation visuelle peut en outre être conçu pour générer une correction cylindrique d'axe de cylindre variable et de puissance variable. Par exemple, le dispositif de compensation visuelle comprend un second élément optique de puissance cylindrique et un troisième élément optique de puissance cylindrique, le second élément optique et le troisième élément optique étant réglables en rotation autour de l'axe optique indépendamment l'un de l'autre.

La lentille de puissance sphérique variable est par exemple une lentille déformable contenant un fluide, ou, autrement dit, une lentille contenant un fluide et une membrane déformable.

L'enceinte est par exemple montée sur un bras articulé par rapport au support orientable autour d'un axe perpendiculaire à l'axe horizontal, ce qui permet de faire varier la convergence de l'axe optique d'observation et d'adapter cette convergence à la vision testée (vision de loin ou vision de près), comme expliqué dans la description qui suit.

L'enceinte peut en outre être montée sur le support orientable à une position réglable selon l'axe horizontal. Précisément, dans l'exemple décrit ci-après, l'enceinte est montée à une position réglable sur le bras articulé.

On peut également prévoir qu'une roue, montée mobile en rotation dans l'enceinte autour d'un axe parallèle à l'axe optique, porte au moins un élément complémentaire destiné à être placé devant l'axe optique. L'élément complémentaire peut être un filtre, un prisme, une ouverture ou un cache.

Le réfracteur peut comprendre des moyens de montage mobile d'un diasporamètre sur la face avant de l'enceinte dans une première position, dans laquelle au moins un prisme du diasporamètre est aligné avec l'axe optique, et dans une seconde position, dans laquelle le diasporamètre laisse libre la première fenêtre optique.

Comme expliqué dans la description qui suit, l'enceinte peut présenter une face inférieure telle que la distance, en projection dans un plan vertical, entre l'axe optique et cette face inférieure soit inférieure ou égale à 30 mm, voire à 20 mm. Ainsi, l'encombrement de l'enceinte est restreint dans la région située sous les yeux de l'utilisateur et l'enceinte ne vient pas interférer avec le visage de l'utilisateur lors de sa rotation autour de l'axe horizontal.

La position du support orientable est par exemple réglable en rotation autour de l'axe horizontal au moyen d'un actionneur. En variante, ce réglage en position pourrait s'effectuer manuellement.

On peut prévoir en outre que la partie fixe comprenne des moyens de positionnement d'une partie de la tête d'un utilisateur (généralement dénommés *"appui front"*).

La partie fixe est par exemple dimensionnée de sorte que ledit axe horizontal passe par les yeux dudit utilisateur (lorsque celui-ci positionne sa tête sur lesdits moyens de positionnement). Plus précisément, la partie fixe est par exemple dimensionnée de sorte que l'axe horizontal passe par le centre de rotation d'au moins un œil dudit utilisateur. Ainsi, le mouvement du support orientable correspondant au mouvement possible de l'œil, par exemple lorsqu'il passe d'une observation en vision de loin à une observation en vision de près.

Le réfracteur comprend au moins un dispositif de capture d'image ayant un axe de prise de vue ; le dispositif de capture d'image peut alors être monté dans le réfracteur de sorte que l'axe de prise de vue soit essentiellement parallèle audit axe horizontal, ce qui permet notamment de surveiller en permanence la situation de l'œil par rapport au dispositif de compensation visuelle.

Le dispositif de capture d'image peut alors être conçu pour mesurer la distance entre l'œil et le dispositif de compensation visuelle. On peut alors prévoir de commander la puissance sphérique du premier élément optique en fonction de la distance mesurée.

L'invention propose également un procédé de mesure de la réfraction au moyen d'un réfracteur comme proposé ci-dessus, comprenant les étapes suivantes :

- inclinaison du support orientable par rapport à la partie fixe ;
- réglage de la puissance sphérique du premier élément optique ;
- mémorisation de la puissance sphérique réglée, par exemple en association avec l'inclinaison précitée.

Comme déjà indiqué, le réglage de la puissance sphérique peut alors être effectué en fonction d'une distance entre un œil d'un utilisateur et le dispositif de compensation visuelle mesurée par un dispositif de capture d'image.

L'invention est définie dans les revendications annexées.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 représente schématiquement les éléments optiques utilisés dans un exemple de mise en œuvre de l'invention ;
- la figure 2 représente un vue en coupe d'un exemple de dispositif de compensation visuelle qui peut être utilisé dans le cadre de l'invention ;
- la figure 3 représente une vue écorchée du dispositif de compensation visuelle de la figure 2 côté lentilles cylindriques ;
- la figure 4 est une vue écorchée du dispositif de compensation visuelle de la figure 2 côté lentille sphérique variable ;
- la figure 5 représente schématiquement un élément de commande du dispositif de compensation visuelle de la figure 2 ;
- la figure 6 est une vue en perspective d'un réfracteur conforme aux enseignements de l'invention dans une première configuration destinée à tester la vision de loin ;
- la figure 7 est une vue en perspective du réfracteur de la figure 6 dans une seconde configuration destinée à tester la vision de près ;
- la figure 8 est une vue de face du réfracteur de la figure 6, vu côté opérateur ;
- la figure 9 est une vue de face du réfracteur de la figure 6, vu côté patient, c'est-à-dire du côté opposé de la vue représentée en figure 8 ;
- la figure 10 est un écorché du support orientable du réfracteur de la figure 6 ;
- la figure 11 est une vue de détail d'un sous-système de compensation visuelle monté sur un bras articulé dans le réfracteur de la figure 6 ;

- la figure 12 est un écorché du sous-système de la figure 11.

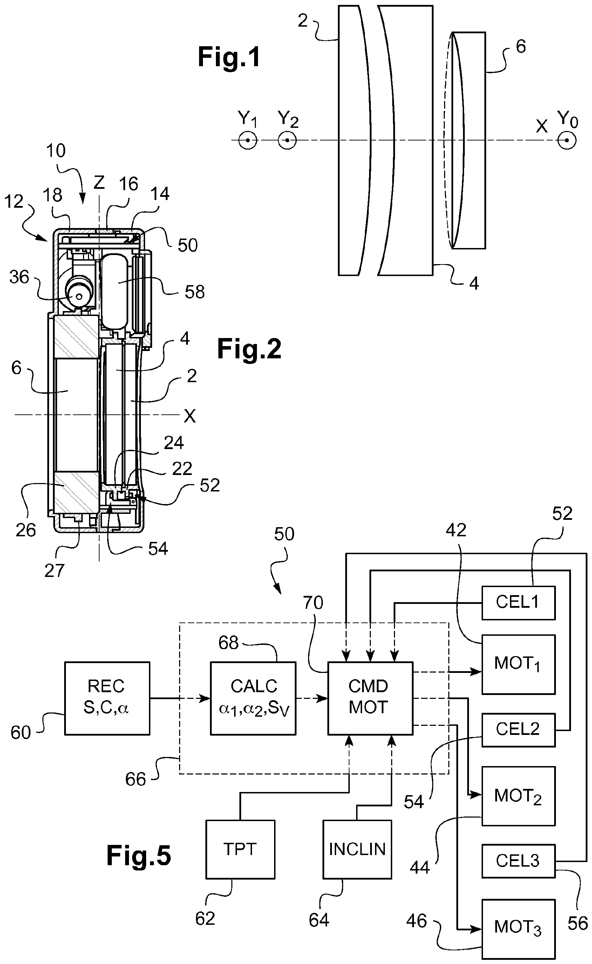

Sur la figure 1 sont schématiquement représentés les éléments optiques principaux d'un exemple de dispositif de compensation visuelle utilisé, comme décrit plus loin, dans un réfracteur conforme aux enseignements de l'invention.

Ces éléments optiques comprennent une lentille plan-cylindre convexe 2, de puissance cylindrique $C_0$, une lentille plan-cylindre concave 4, de puissance cylindrique négative $-C_0$, et une lentille 6 de puissance sphérique variable $S_V$.

La valeur absolue (ou module), ici $C_0$, de la puissance cylindrique (ici $-C_0$) de la lentille plan-cylindre concave 4 est donc égale à la valeur absolue ($C_0$) (ou module) de la puissance cylindrique ($C_0$) de la lentille plan-cylindre convexe 2.

On pourrait prévoir en variante que les puissances cylindriques respectives de la lentille plan-cylindre concave 4 et de la lentille plan-cylindre convexe 2 soient (légèrement) différentes en valeur absolue, mais soient en tout état de cause telles que la puissance cylindrique résultante, générée par la combinaison de ces deux lentilles, ait une valeur négligeable (par exemple inférieur à 0,1 dioptrie en valeur absolue) dans au moins une position relative de ces deux lentilles.

Les trois lentilles 2, 4, 6 sont placées sur le même axe optique X. Précisément, chacune des trois lentilles 2, 4, 6 a une forme extérieure généralement cylindrique, centrée sur l'axe optique X. Dans l'exemple décrit ici, les lentilles 2, 4, 6 ont respectivement les diamètres (mesurant leur encombrement) suivants : 25 mm, 25 mm, 20 mm.

On remarque de ce fait qu'il est préférable d'utiliser ce dispositif de compensation visuelle 10 en positionnant l'œil du patient du côté de la lentille de puissance sphérique variable 6 de sorte que les lentilles de puissance cylindriques 2, 4, de plus grand diamètre, ne viennent pas limiter le champ de vision défini par la lentille de puissance sphérique variable 6, qui est lui-même large du fait de la proximité de l'œil du patient.

Chacune des trois lentilles 2, 4, 6 comporte une première face plane, perpendiculaire à l'axe optique X, et une seconde face, opposée à la première face et optiquement active :

- la face optiquement active de la lentille 2 est de forme cylindrique convexe (l'axe $Y_1$ du cylindre définissant cette face étant perpendiculaire à l'axe optique X) ;
- la face optiquement active de la lentille 4 est de forme cylindrique concave (l'axe $Y_2$ du cylindre définissant cette face étant perpendiculaire à l'axe optique X) ;
- la face optiquement active de la lentille 6 de puissance sphérique variable $S_V$ est déformable et peut ainsi prendre une forme sphérique convexe (comme illustré en pointillés sur la figure 1), une forme plane ou une forme sphérique concave (comme illustré en trait plein).

La lentille 6 de puissance sphérique variable $S_V$ est par exemple une lentille du type décrit dans le document EP 2 034 338. Une telle lentille comprend une cavité fermée par une membrane déformable transparente et une paroi plane transparente mobile ; la cavité contient un liquide transparent de volume constant qui est plus ou moins contraint par la face mobile, afin de déformer la membrane qui est de ce fait soit une surface concave sphérique, soit une surface plane, soit une surface convexe sphérique. Dans la lentille utilisée, une transformation de mouvement réalisée par un système vis écrou permet d'assurer la transformation de mouvement translation - rotation. Ainsi, une rotation d'une bague montée sur un boîtier 26 entraîne en translation une pièce de la lentille 6, ce qui provoque la déformation susmentionnée de la membrane transparente comme expliqué par exemple dans le document EP 2 034 338 précité. On peut ainsi faire varier continûment la puissance sphérique $S_V$ par action mécanique sur la lentille 6. Dans l'exemple décrit ici, la lentille 6 a une focale variable entre -40 mm et 40 mm, soit une puissance sphérique $S_V$ variable entre -25D et 25D (D étant la dioptrie, unité de mesure de la vergence, inverse de la focale exprimée en mètres).

Par ailleurs, les lentilles plan-cylindre 2, 4 ont respectivement comme déjà indiqué une puissance cylindrique $-C_0$ et $C_0$, ici avec $C_0 = 5D$.

Comme expliqué plus en détail dans la suite, la lentille plan-cylindre concave 4 et la lentille plan-cylindre convexe 2 sont montées en rotation autour de l'axe X (rotation centrée sur l'axe X).

L'axe $Y_1$ du cylindre convexe formé sur la face optiquement active de la lentille plan-cylindre convexe 2 peut ainsi former un angle variable $\alpha_1$ avec un axe de référence $Y_0$ (fixe et perpendiculaire à l'axe optique X).

De même, l'axe $Y_2$ du cylindre concave formé sur la face optiquement active de la lentille plan-cylindre concave 4 peut former un angle variable $\alpha_2$ avec l'axe de référence $Y_0$.

Par calcul de la vergence sur les différents méridiens, on obtient les formules suivantes pour la puissance sphérique S, la puissance cylindrique C et l'angle d'astigmatisme $\alpha$ du sous-ensemble optique formé des trois éléments optiques 2, 4, 6 qui vient d'être décrit :

$$\tan 2\alpha = \frac{\sin 2\alpha_2 - \sin 2\alpha_1}{\cos 2\alpha_2 - \cos 2\alpha_1} = -\frac{\cos(\alpha_1 + \alpha_2)}{\sin(\alpha_1 + \alpha_2)} \text{ (formule 1)}$$

$$C = C_0 \left( \cos 2(\alpha - \alpha_2) - \cos 2(\alpha - \alpha_1) \right) \text{ (formule 2)}$$

$$S = S_V - \frac{C}{2} \text{ . (formule 3).}$$

On remarque que le terme (-C/2) dans la formule 3 correspond à une puissance sphérique générée par la résultante des 2 lentilles à puissance cylindrique.

En pilotant la position en rotation de la lentille plan-cylindre convexe 2 et la position en rotation de la lentille plan-cylindre concave 4, indépendamment l'une de l'autre, comme décrit ci-après, on peut faire varier indépendamment chacun des angles $\alpha_1$, $\alpha_2$ de 0° à 360° et ainsi obtenir une puissance cylindrique C réglable entre -2.$C_0$ et 2.$C_0$ (soit ici entre -10D et 10D), et pour n'importe quel angle d'astigmatisme réglable entre 0° et 360° obtenu par une commande simultanée des deux lentilles. Comme l'indique la formule numéro 3, la résultante de puissance sphérique induite par la résultante de l'orientation des 2 lentilles cylindriques est compensée à l'aide de la lentille sphérique de puissance variable.

Par ailleurs, en faisant varier la puissance sphérique $S_V$ de la lentille sphérique 6, on peut régler la puissance sphérique S du sous-ensemble formé des trois lentilles 2, 4, 6.

Selon une variante envisageable, les lentilles à puissance cylindrique fixe pourraient avoir la même puissance cylindrique $C_0$ (positive ou négative) : il pourrait s'agir de deux lentilles plan-cylindre convexe, éventuellement identiques, ou, en alternative, de deux lentilles plan-cylindre concave, éventuellement identiques.

En effet, dans ce cas, la puissance sphérique S, la puissance cylindrique C et l'angle d'astigmatisme $\alpha$ du sous-ensemble formé de ces deux lentilles et d'une lentille à puissance sphérique variable sont donnés par les formules suivantes :

$$\tan 2\alpha = \frac{\sin 2\alpha_2 + \sin 2\alpha_1}{\cos 2\alpha_2 + \cos 2\alpha_1} \text{ (formule 4)}$$

$$C = C_0 \left( \cos 2(\alpha - \alpha_2) + \cos 2(\alpha - \alpha_1) \right) \text{ (formule 5)}$$

$$S = S_V + C_0 - \frac{C}{2} \text{ . (formule 6)}$$

Le terme $C_0$ - C/2 correspond à la puissance sphérique induite par la combinaison des deux lentilles à puissance cylindrique.

On peut donc également dans ce cas régler la puissance sphérique S, la puissance cylindrique C et l'angle d'astigmatisme $\alpha$, en particulier de sorte que la puissance cylindrique C soit nulle, en entraînant en rotation les lentilles à puissance cylindrique (indépendamment l'une de l'autre) et en faisant varier la puissance sphérique de la lentille à puissance sphérique variable.

Un exemple de dispositif de compensation visuelle 10 qui utilise les éléments optiques qui viennent d'être décrits est représenté en figure 2.

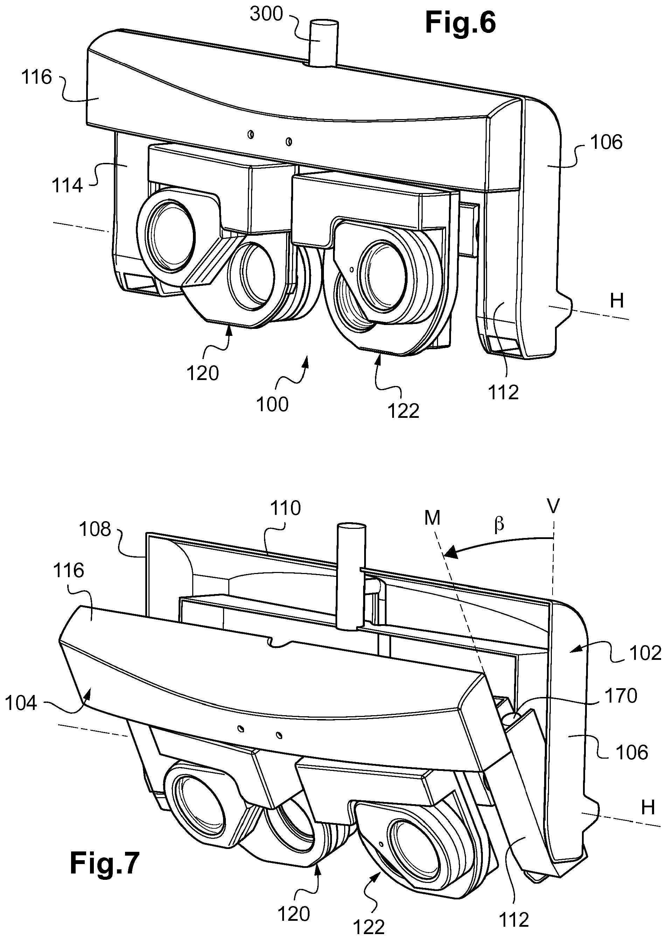

On utilisera parfois dans la description qui suit, afin de clarifier l'explication, des termes, comme "*supérieur*" ou "*inférieur*", qui définissent une orientation dans les figures 2, 3 et 4. On comprend que cette orientation n'est pas nécessairement applicable à l'utilisation qui pourra être faite du dispositif décrit, en particulier celle des figures 6 à 12.

Le dispositif de compensation visuelle 10 comprend un boîtier 12 formé d'une première partie 14, d'une seconde partie 16 et d'une troisième partie 18, qui s'étendent successivement selon l'axe optique X et sont assemblées deux à deux au niveau de plans perpendiculaires à l'axe optique X.

Une première roue dentée 22 est montée en rotation centrée sur l'axe optique X dans la première partie 14 du boîtier 12 et porte en son centre, dans une ouverture prévue à cet effet, la lentille plan-cylindre convexe 2. La première roue dentée 22 et la lentille plan-cylindre convexe 2 sont coaxiales ; autrement dit, en section dans un plan perpendiculaire à l'axe optique X, la circonférence extérieure de la première roue dentée 22 et la circonférence de la lentille plan-cylindre convexe 2 forment des cercles concentriques centrés sur l'axe optique X.

De même, une seconde roue dentée 24 est montée en rotation centrée sur l'axe optique X dans la seconde partie 16 du boîtier 12 et porte en son centre, dans une ouverture prévue à cet effet, la lentille plan-cylindre concave 4.

La seconde roue dentée 24 et la lentille plan-cylindre concave 4 sont coaxiales ; autrement dit, en section dans un plan perpendiculaire à l'axe optique X, la circonférence extérieure de la seconde roue dentée 24 et la circonférence de la lentille plan-cylindre concave 4 forment des cercles concentriques centrés sur l'axe optique X.

Une troisième roue dentée 27 est montée en rotation centrée sur l'axe optique X dans la troisième partie 18 du boîtier 12. La troisième roue dentée 27 est solidaire de la bague pourvue sur la circonférence du boîtier 26 qui porte la lentille 6 de puissance sphérique variable et permettant la commande de la puissance sphérique $S_V$. Le boîtier 26 de la lentille 6 de puissance sphérique variable est monté dans la troisième partie 18 du boîtier 12.

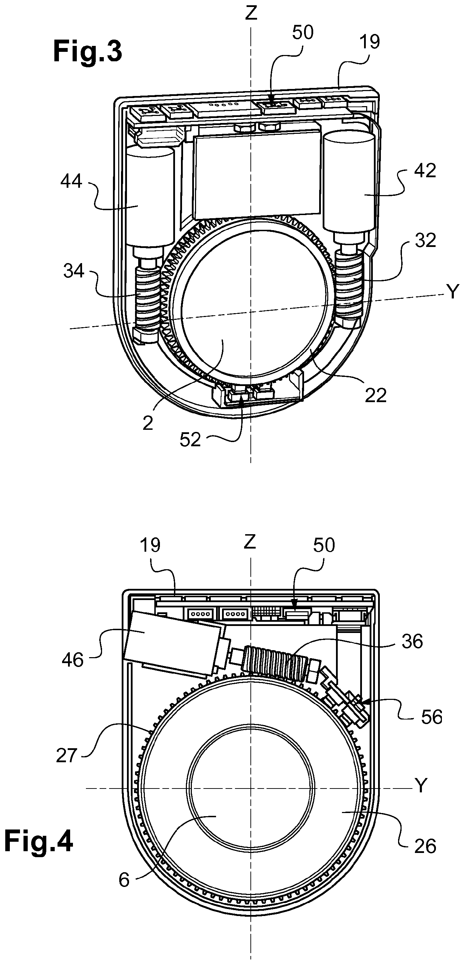

Comme bien visible en figure 3, la première roue dentée 22 est entraînée en rotation (autour de l'axe optique X) au moyen d'un premier moteur 42 dont un axe d'entraînement porte une première vis sans fin 32 qui engrène sur la première roue dentée 22. Le premier moteur 42 est par exemple monté dans la première partie 14 du boîtier 12.

La position courante de la première roue dentée 22 est surveillée par une première cellule optique 52.

De même, la seconde roue dentée 24 est entraînée en rotation autour de l'axe optique X au moyen d'un second moteur 44 dont un axe d'entraînement porte une seconde vis sans fin 34 qui engrène sur la seconde roue dentée 24. Le second moteur 44 est par exemple monté dans la seconde partie 16 du boîtier 12.

La position courante de la seconde roue dentée 24 est surveillée par une seconde cellule optique 54.

Comme représenté sur la figure 4, la troisième roue dentée 27 est quant à elle entraînée en rotation (autour de l'axe optique X) au moyen d'un troisième moteur 46 qui présente un axe d'entraînement sur lequel est monté une troisième vis sans fin 36 qui engrène avec la troisième roue dentée 27. Le troisième moteur 46 est par exemple monté dans la troisième partie 18 du boîtier 12.

La position courante de la troisième roue dentée 27 est surveillée par une troisième cellule optique 56.

Chaque cellule optique 52, 54, 56 est par exemple formée d'un couple d'éléments comprenant au moins un capteur optique ; l'autre élément du couple est par exemple un émetteur optique (ou, en variante, un élément réfléchissant, auquel cas un émetteur optique est associé au capteur optique).

Les premier, second et troisième moteurs 42, 44, 46 sont par exemple des moteurs pas à pas, d'une résolution de 20 pas/tour, pilotés ici en 8ème de pas (ci-après micro-pas). En variante, ces moteurs pourraient être pilotés en 16ème de pas.

Grâce à la construction décrite ci-dessus, les éléments optiques (c'est-à-dire le premier élément optique, le second élément optique et la lentille) sont montés (dans le dispositif de compensation visuelle) de manière à conserver (chacun) leur position de consigne respective (même) sans alimentation électrique.

Le volume interne du boîtier 12 (comme d'ailleurs le volume interne de chacune des première, seconde et troisième parties 14, 16, 18 de la même manière) peut être subdivisé en un espace de réception des moteurs 42, 44, 46 (région supérieure du boîtier 12 sur les figures 2, 3 et 4) et un espace de réception des éléments optiques 2, 4, 6 (région inférieure du boîtier 12 sur les figures 2, 3 et 4).

L'espace de réception des moteurs 42, 44, 46 a une forme essentiellement parallélépipédique, ouverte (vers le bas sur les figures) en direction de l'espace de réception des éléments optiques 2, 4, 6 et fermé à l'opposé (vers le haut sur les figures) par une face supérieure 19 du boîtier 12 (la face supérieure 19 du boîtier 12 étant formée par l'assemblage de faces supérieures respectives des première, seconde et troisième parties 14, 16, 18 du boîtier 12).

La disposition des moteurs 42 44 et 46 est telle qu'elle permet de bénéficier d'une géométrie circulaire sur 180° centrée sur l'axe optique au plus proche du rayon utile des lentilles.

L'espace de réception des éléments optiques 2, 4, 6 présente, à l'opposé de l'espace de réception des moteurs, une forme cylindrique (délimitée par les parois du boîtier 12) qui épouse celle de la troisième roue dentée 27 sur la moitié de la circonférence de celle-ci.

Autrement dit, le boîtier 12 (et par conséquent chacune des première, seconde et troisième parties 14, 16, 18 du boîtier 12) a, au niveau de l'espace de réception des éléments optiques 2, 4, 6, une forme cylindrique de diamètre (perpendiculairement à l'axe optique X) du même ordre que, et légèrement supérieur à, celui de la troisième roue dentée 27.

Les diamètres respectifs des roues dentées 22, 24, 27 sont adaptés de manière à favoriser la conservation du champ en dépit de l'épaisseur du sous-ensemble optique.

Le premier moteur 42 et la première vis sans fin 32 s'étendent dans le boîtier 12 selon une direction Z perpendiculaire à la face supérieure du boîtier 12 (et donc notamment perpendiculaire à l'axe optique X) de telle sorte que le premier moteur 42 est logé dans l'espace de réception des moteurs tandis que la première vis sans fin 32 s'étend dans l'espace de réception des éléments optiques.

Le second moteur 44 et la seconde vis sans fin 34 s'étendent quant à eux dans le boîtier 12 selon la même direction, mais à l'opposé du premier moteur 42 et de la première vis sans fin 34 par rapport aux lentilles de puissance cylindrique 2, 4. Le second moteur 44 est logé dans l'espace de réception des moteurs tandis que la seconde vis sans fin 34 s'étend dans l'espace de réception des éléments optiques.

On remarque qu'ainsi la première vis sans fin 32 et la seconde vis sans fin 34 sont situées de part et d'autre de l'ensemble formé par la première roue dentée 22 et la seconde roue dentée 24, et que l'encombrement latéral (selon un axe Y perpendiculaire aux axes X et Z précités) de ces différentes pièces (première vis sans fin 32, seconde vis sans fin 34, première ou seconde roue dentée 22, 24) est inférieur au diamètre de la troisième roue dentée 27 de sorte que les première et seconde vis sans fin 32, 34 contiennent dans l'espace de réception des éléments optiques sans nécessiter d'excroissance pour les accueillir.

Par ailleurs, les premier et second moteurs 42, 44 ont chacun un encombrement selon l'axe optique X supérieur à celui de chacune des première et seconde roues dentées 22, 24, et même supérieur à celui de chacune des première et seconde parties 14, 16 de boîtier 12. Toutefois, du fait que ces premier et second moteurs 42, 44 sont placés comme il vient d'être indiqué de chaque côté du boîtier 12 (par rapport à l'axe Z), ils peuvent chacun occuper un espace qui s'étend selon l'axe optique X au droit de la première partie 14 et de la seconde partie 16 du boîtier 12.

Par exemple, chacun des premier et second moteurs 42, 44 a un encombrement latéral (diamètre externe du moteur) compris entre 6 et 12, par exemple 10 mm, tandis que les première et seconde roues dentées 22, 24 ont chacune une épaisseur (encombrement selon l'axe X) compris entre 1 et 4, par exemple 2,5 mm.

Le troisième moteur 46 et la troisième vis sans fin 36 sont en revanche situés dans l'espace de réception des moteurs, dans la région qui s'étend selon l'axe X au droit de la troisième partie 18 du boîtier 12. Ainsi, la troisième vis sans fin 36 engrène la troisième roue dentée 27 dans une partie supérieure de celle-ci, ce qui permet au boîtier 12 d'épouser la forme du boîtier 12 dans la partie inférieure de la troisième roue dentée 27, comme déjà indiqué.

Dans l'exemple décrit, comme visible en figure 4, l'axe du troisième moteur 46 et de la troisième vis sans fin 36 est légèrement incliné par rapport à la face supérieure du boîtier 12 (précisément par rapport à l'axe Y précité).

On prévoit par exemple que l'épaisseur de la troisième roue dentée 27 est comprise entre 0,3 mm et 2 mm.

Cette disposition des différents éléments permet d'obtenir un boîtier relativement fin, ayant typiquement une épaisseur comprise entre 15 et 20 mm.

Le boîtier 12 comprend également, par exemple dans la région supérieure de l'espace de réception des moteurs, un élément de commande 50, formé ici de plusieurs circuits intégrés portés par un circuit imprimé commun.

Par ailleurs un dispositif de stockage d'énergie électrique de type batterie 58 (ou, en variante, une super capacité) est prévu pour rendre l'appareil autonome. On prévoit par exemple également des éléments de recharge sans contact du dispositif de stockage d'énergie 58. La batterie 58 permet notamment l'alimentation électrique des moteurs 42, 44, 46 et de l'élément de commande 50.

Les éléments principaux d'un tel élément de commande 50, ainsi que leur connexion aux moteurs 42, 44, 46 précités et aux cellules optiques 52, 54, 56 précitées, sont représentés schématiquement en figure 5.

L'élément de commande 50 comprend un module de réception 60 conçu pour recevoir, ici à travers une liaison sans fil, les informations de consigne, c'est-à-dire des informations indicatives des valeurs souhaitées par l'utilisateur pour la puissance sphérique S, la puissance cylindrique C et l'angle d'astigmatisme $\alpha$ qui définissent la compensation générée par le sous-ensemble optique formé des éléments optiques 2, 4, 6.

Le module de réception 60 est par exemple un module de réception infrarouge qui reçoit ces informations de consigne d'une télécommande à émission infrarouge manipulée par l'utilisateur. En variante, on pourrait prévoir que ces informations de consigne soit reçues d'un ordinateur personnel via une liaison sans fil, par exemple un réseau local sans fil ; l'utilisateur pourrait dans ce cas choisir des valeurs de puissance sphérique S, de puissance cylindrique C et d'angle d'astigmatisme $\alpha$ pour le dispositif de compensation visuelle par sélection interactive sur l'ordinateur.

Dans l'exemple d'application décrit ci-après en référence aux figures 6 à 12, les informations de consigne peuvent par exemple être reçues de la carte électronique de commande 160 via le dispositif émetteur/récepteur 156.

Le module de réception 60 transmet les informations de consigne S, C, $\alpha$ reçues à un calculateur 66 (constitué par exemple d'un processeur exécutant un programme d'ordinateur de manière à mettre en œuvre les fonctions du calculateur décrites ci-après), précisément à un module de calcul 68 mis en œuvre par ce calculateur 66.

Le module de calcul 68 calcule les valeurs des angles $\alpha_1$, $\alpha_2$ et la valeur de puissance sphérique $S_V$ nécessaires afin d'obtenir les valeurs de consignes S, C, $\alpha$ reçues en entrée, sur la base des formules exposées plus haut. Dans le cas où les lentilles plan-cylindre 2 et 4 ont respectivement une puissance cylindrique - $C_0$ et $C_0$, on utilise par exemple les formules suivantes :

$$\begin{cases} \alpha_1 = \alpha - \dfrac{1}{2}\arcsin\left(\dfrac{C}{2C_0}\right) + \dfrac{\pi}{4} \\[2mm] \alpha_2 = \alpha + \dfrac{1}{2}\arcsin\left(\dfrac{C}{2C_0}\right) + \dfrac{\pi}{4} \end{cases}$$

$$S_V = S + \frac{C}{2}$$

Le calculateur 66 met également en œuvre un module de commande 70 qui reçoit en entrée les valeurs d'angle $\alpha_1$, $\alpha_2$ et de puissance sphérique $S_V$ calculées par le module de calcul 68 et émet des signaux de commande à destination des moteurs 42, 44, 46 afin de commander chacun des moteurs 42, 44, 46 indépendamment des autres de manière à obtenir des positionnements respectifs des roues dentées 22, 24, 27 qui permettent d'obtenir les valeurs souhaitées :

- le module de commande 70 commande le premier moteur 42 de manière à faire tourner la première roue dentée 22 autour de l'axe optique X jusqu'à la position où l'axe $Y_1$ de la surface cylindrique optiquement active de la lentille plan-cylindre convexe 2 (portée par la première roue dentée 22) forme un angle $\alpha_1$ avec la direction de référence $Y_0$ ;
- le module de commande 70 commande le second moteur 44 de manière à faire tourner la seconde roue dentée 24 autour de l'axe optique X jusqu'à la position où l'axe $Y_2$ de la surface cylindrique optiquement active de la lentille plan-cylindre concave 4 (portée par la seconde roue dentée 24) forme un angle $\alpha_2$ avec la direction de référence $Y_0$ ;
- le module de commande 70 commande le troisième moteur 46 de manière à faire tourner la troisième roue dentée 27 autour de l'axe optique X jusqu'à la position où la bague de commande de la puissance sphérique variable commande la puissance sphérique $S_V$ calculée par le module de calcul 68.

La position de chaque roue dentée 22, 24, 27 est connue à chaque instant respectivement grâce aux cellules optiques 52, 54, 56 qui mesurent chacune, sur la roue dentée à laquelle chacune est associée, le nombre de dents ayant traversé la cellule optique par rapport à un point de référence sur la circonférence de la roue concernée (par exemple dépourvu de dent).

Dans l'exemple décrit ici, l'ensemble premier moteur 42-première vis sans fin 32-première roue dentée 22, comme l'ensemble second moteur 44-seconde vis sans fin 34-seconde roue dentée 24, génère une démultiplication telle qu'un tour de roue dentée 22, 24 correspond à 15040 micro-pas du moteur associé 42, 44. La résolution (angle de rotation des roues dentées 22, 24 pour un micro-pas) est donc de 0,024° pour les angles $\alpha_1$ et $\alpha_2$.

L'ensemble troisième moteur 46-troisième vis sans fin 36-troisième roue dentée 46 génère quant à lui une démultiplication de 16640 micro-pas par tour. La bague de commande de la puissance sphérique variable est réglable sur une plage angulaire de 120° (ce qui correspond donc à 5547 micro-pas) afin d'obtenir la variation de puissance sphérique de -25D à 25D (soit une plage de variation de 50D). La résolution (variation de puissance sphérique $S_V$ pour un micro-pas) est donc de 0,009D.

On peut prévoir que, lors du passage de valeurs de consigne initiales $\alpha_1$, $\alpha_2$, $S_V$ à de nouvelles valeurs de consigne $\alpha'_1$, $\alpha'_2$, $S'_V$, chacun des premier, second et troisième moteurs 42, 44, 46 soient actionnés pendant une même durée T (en secondes), qui peut dépendre éventuellement de l'amplitude de l'un des changements de consigne (par exemple de la variation, en valeur absolue, de puissance sphérique $| S'_V - S_V |$, où $| x |$ est la valeur absolue de x).

Pour ce faire, le calculateur 66 détermine par exemple le nombre $p_1$ de micro-pas du moteur 42 permettant le passage de l'angle $\alpha_1$ à l'angle $\alpha'_1$, le nombre $p_2$ de micro-pas du moteur 44 permettant le passage de l'angle $\alpha_2$ à l'angle $\alpha'_2$ et le nombre $p_3$ de micro-pas du moteur 46 permettant le passage de la puissance sphérique $S_V$ à la puissance sphérique $S'_V$. Le calculateur 66 commande alors la rotation du moteur 42 à une vitesse de $p_1$/T micro-pas par seconde, la rotation du moteur 44 à une vitesse de $p_2$/T micro-pas par seconde et la rotation du moteur 46 à une vitesse de $p_3$/T micro-pas par seconde.

L'élément de commande 50 comprend également un capteur de température 62, qui délivre une information de température ambiante mesurée, et un inclinomètre 64, par exemple réalisé sous forme d'un accéléromètre et qui délivre une information d'orientation du dispositif de compensation visuelle 10, par exemple par rapport à la verticale. Dans l'application décrite ci-après en référence aux figures 6 à 12, l'information d'orientation peut être utilisée afin de déterminer la configuration du réfracteur et ainsi le type de mesure d'acuité couramment effectué (vision de loin, vision intermédiaire ou vision de près).

Le calculateur 66 reçoit l'information de température en provenance du capteur de température 62 et l'information d'orientation en provenance de l'inclinomètre 64 et utilise l'une au moins de ces informations dans le cadre de la détermination des commandes à envoyer aux moteurs 42, 44, 46.

Dans l'exemple décrit, le module de commande 70 utilise l'information de température afin de compenser les variations de puissance sphérique de la lentille 6 dues à la température (qui sont de l'ordre de 0,06D/°C dans l'exemple décrit) et l'information d'orientation afin de compenser les perturbations éventuelles du système d'entraînement (moteurs, vis sans fin, roues dentées) dues à des changements d'orientation du dispositif de compensation visuelle 10.

Contrairement au cas de la description des figures 2 à 4 ci-dessus, la description qui suit des figures 6 à 12 fait référence à des directions (horizontale et verticale notamment) et à des positionnements relatifs ("*inférieur*" ou "*supérieur*") qui correspondent à l'utilisation classique du réfracteur pour la mesure d'acuité visuelle d'un patient.

Les figures 6 et 7 représentent en perspective un réfracteur 100 conforme aux enseignements de l'invention, respectivement dans une première configuration destinée à tester la vision de loin et dans une seconde configuration destinée à tester la vision de près, étant entendu que le réfracteur peut également prendre toutes les positions intermédiaires.

Le réfracteur 100 comprend une partie fixe 102, destinée à être montée sur un support de tête de réfraction, et une partie mobile 104 formant support orientable, montée sur la partie fixe 102 avec possibilité de rotation autour d'un axe horizontal H, par exemple sur une plage angulaire donnée.

La partie fixe 102 a ici une forme de portique et comprend deux montants parallèles verticaux 106, 108 et une poutre horizontale 110 joignant les deux montants 106, 108 dans leur partie supérieure et au niveau de laquelle la partie fixe 102 peut être montée sur le support de tête de réfraction, dont une partie 300 est visible sur les figures 6 et 7.

Dans le mode de réalisation décrit, le support orientable 104 présente également une forme de portique et comprend ainsi deux montants parallèles 112, 114 et une traverse 116 qui joint les deux montants 112, 114 dans leur partie supérieure.

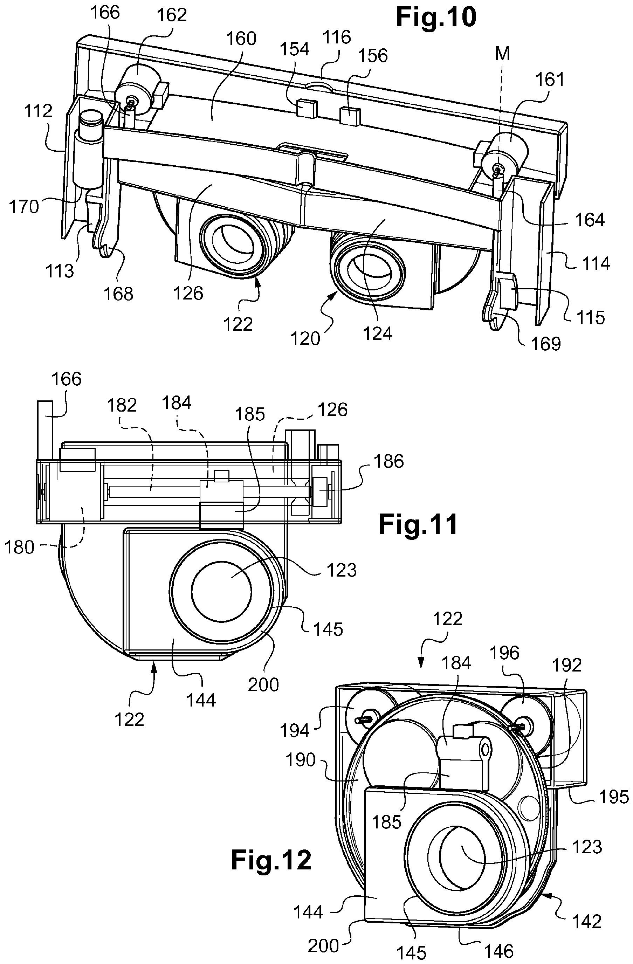

Le montant 106 de la partie fixe 102 et le montant 112 du support orientable 104 comprennent des moyens complémentaires de guidage en rotation autour de l'axe horizontal H (voir la référence 113 en figure 10), situés ici dans la région inférieure de ces montants 106, 112.

De même, le montant 108 de la partie fixe 102 et le montant 114 du support orientable 104 comprennent des moyens complémentaires de guidage en rotation autour de l'axe horizontal H (voir la référence 115 en figure 10), situés ici dans la région inférieure de ces montants 108, 114.

Ainsi, le support orientable 104 est déplaçable, par rapport à la partie fixe 102 et autour de l'axe horizontal H, entre une position dans laquelle ses montants 112, 114 sont verticaux (illustrée en figure 6) et une position dans laquelle ses montants 112, 114 sont inclinés d'un angle d'inclinaison $\beta$ par rapport à la verticale V (illustrée en figure 7), avec ici $\beta = 30°$.

Le support orientable 104 peut être positionné dans une ou plusieurs position(s) intermédiaire(s) entre ces deux positions.

La mise en mouvement du support orientable 104 par rapport à la partie fixe 102 est ici motorisée : une vis sans fin entraînée par un moteur 170 (bien visible en figure 10) solidaire du support orientable 104 engrène par exemple sur une portion de pignon solidaire de la partie fixe 102.

En variante, le support orientable 104 pourrait être déplacé manuellement par rapport à la partie fixe 102 ; on peut alors prévoir des moyens mécaniques permettant d'arrêter la rotation dans une ou plusieurs position(s) du support orientable 104 par rapport à la partie fixe 102.

Les montants 106, 108 de la partie fixe 102 ont ici sensiblement la même forme extérieure que les montants 112, 114 du support orientable 104 respectivement ; le montant 106 de la partie fixe 102 est en outre placé au droit du montant 112 du support orientable 104 et le montant 108 de la partie fixe 102 est situé au droit du montant 114 du support orientable 104. Par ailleurs, la poutre 110 de la partie fixe 102 est située au droit de la traverse 116.

On prévoit ici au surplus que les éléments structurels de la partie fixe 102 (c'est-à-dire les montants 106, 108 et la poutre 110) soient creux afin d'accueillir les éléments correspondants du support orientable 104 (soit respectivement les montants 112, 114 et la traverse 116) lorsque ce dernier est dans sa position verticale, comme illustré en figure 6.

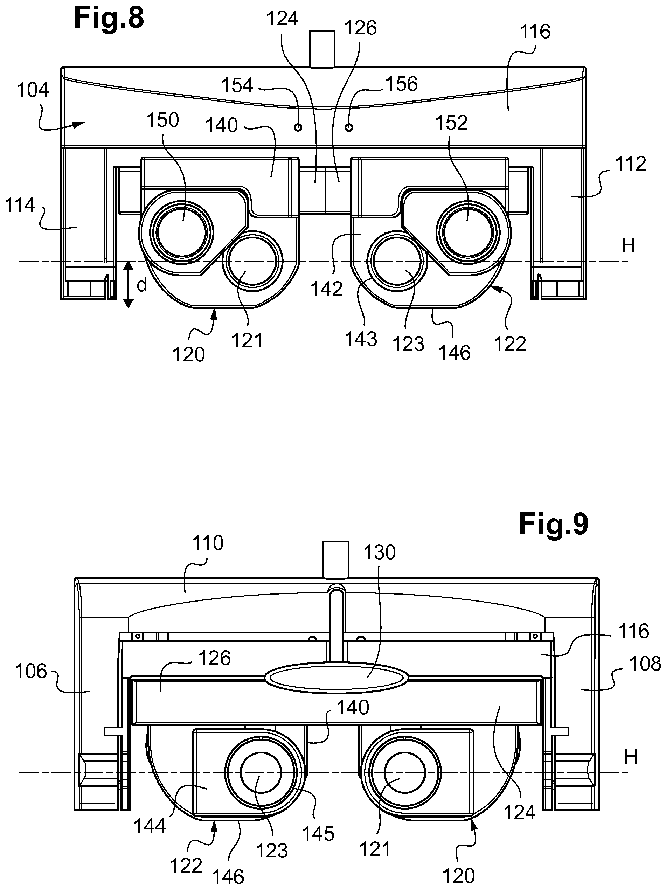

Les figures 8 et 9 sont des vues de face du réfracteur qui vient d'être présenté, respectivement vu côté opérateur et vu côté patient.

Comme bien visible sur ces figures, le réfracteur 100 comprend un premier sous-système de compensation visuelle 120 présentant un oculaire 121 destiné à l'œil droit du patient et un second sous-système 122 présentant un oculaire 123 destiné à l'œil gauche du patient.

Le premier sous-système 120 est monté (avec une possibilité de déplacement en translation horizontale comme décrit ci-après en référence à la figure 11) sur un premier bras articulé 124 qui s'étend parallèlement à la traverse 116, approximativement sur la moitié de la longueur de la traverse 116.

Le premier bras articulé 124 est monté sur la traverse 116, dans la région d'extrémité de celle-ci, c'est-à-dire à proximité du montant 114, avec une possibilité de rotation autour d'un axe M qui s'étend dans la direction générale d'extension du montant 114, comme expliqué plus loin en référence à la figure 10.

Le premier bras articulé 124 est monté sous la traverse 116 et le premier sous-système 120 est monté sur le premier bras articulé 124 de manière à s'étendre, pour l'essentiel, sous le premier bras articulé 124. En particulier, l'oculaire 121 du premier sous-système 120 est situé dans son intégralité sous le premier bras articulé 124.

De manière similaire, le second sous-système 122 est monté (avec une possibilité de déplacement en translation horizontale comme décrit ci-après en référence à la figure 11) sur un second bras articulé 126 qui s'étend parallèlement à la traverse 116, approximativement sur l'autre moitié de la longueur de la traverse 116.

Le second bras articulé 126 est monté sur la traverse 116, dans la région d'extrémité de celle-ci (opposée à la région de montage du premier bras articulé 124), c'est-à-dire à proximité du montant 112, avec une possibilité de rotation autour d'un axe qui s'étend dans la direction générale d'extension du montant 112, comme expliqué plus loin en référence à la figure 10.

Le second bras articulé 124 est monté sous la traverse 116 et le second sous-système 120 est monté sur le second bras articulé 126 de manière à s'étendre, pour l'essentiel, sous le second bras articulé 126. En particulier, l'oculaire 123 du second sous-système 122 est situé dans son intégralité sous le second bras articulé 126.

Plus précisément, les centres respectifs des oculaires 121, 123 des premier et second sous-systèmes 120, 122 sont situés dans le plan horizontal contenant l'axe horizontal H de rotation du support orientable 104 par rapport à la partie fixe 102.

Comme bien visible en figure 9, la partie fixe 102 porte également des moyens de positionnement de la tête du patient 130, généralement dénommés *"appui front",* éventuellement à une position réglable en translation selon la direction verticale (c'est-à-dire parallèlement à l'axe vertical V).

On propose ici que le réfracteur soit dimensionné de telle sorte que l'axe horizontal H passe par les centres de rotation des yeux du patient.

Ainsi, lorsque le patient positionne sa tête contre les moyens de positionnement avec ses yeux en face des oculaires 121, 123, les sous-systèmes 120, 122 ont un mouvement très limité au niveau des oculaires 121, 123 lors de l'inclinaison du support orientable 104 par rapport à la partie fixe 102, qui suit en outre le regard du patient (notamment lorsque celui-ci baisse le regard en vision de près).

Chaque sous-système 120, 122 comprend plusieurs éléments protégés par une enceinte 140 formée notamment d'une face avant 142, d'une face arrière 144 et d'une face inférieure 146. Chaque sous-système 120, 122 comprend notamment un dispositif de compensation visuelle 200 tel que décrit ci-dessus en référence aux figures 1 à 5.

La face avant 142 et la face arrière 144 présentent chacune une fenêtre optique 143, 145 qui délimite (dans chaque face 142, 144) l'oculaire 121, 123 du sous-système concerné 120, 122.

Le sous-système 122 est décrit ci-dessous de manière détaillée en référence à la figure 12. Le sous-système 120 est construit de manière similaire (les deux sous-systèmes 120, 122 étant symétriques par rapport à un plan vertical passant par le milieu de la traverse 116).

Grâce à l'encombrement limité du dispositif de compensation visuelle 200 décrit ci-dessus en référence aux figures 1 à 5, la face inférieure 146 de chaque sous-système 120, 122 est proche de l'axe optique du dispositif de compensation visuelle concerné (autrement dit du centre de l'oculaire 121, 123 concerné). Ainsi, du fait de la construction adoptée comme indiqué ci-dessus (axe de rotation H au niveau des centres des oculaires 121, 123), la face inférieure 146 de chaque sous-système 120, 122 est proche (par exemple située à une distance d inférieure ou égale à 30 mm ou moins, de préférence inférieure ou égale à 20 mm - ici égale à 20 mm, en projection dans un plan vertical) de l'axe de rotation H du support orientable 104 par rapport à la partie fixe 102.

De ce fait, le support orientable 104, et en particulier les sous-systèmes 120, 122, ne viennent pas interférer avec le visage de l'utilisateur lorsque le support orientable 104 est déplacé en rotation autour de l'axe horizontal H pour passer de la configuration adaptée à la mesure de la vision de loin (figure 6) à la configuration adaptée à la mesure de la vision de près (figure 7).

Chaque sous-ensemble 120, 122 comprend également des moyens de positionnement d'un diasporamètre 150, 152 mobile entre deux positions distinctes sur la face avant 142 : dans une première position, les prismes du diasporamètre sont alignés avec le centre de l'oculaire 121, 123 concerné, c'est-à-dire avec l'axe optique du dispositif de compensation visuelle concerné ; dans la seconde position, le diasporamètre est positionné en dehors de l'oculaire 121, 123 concerné et laisse libre la fenêtre optique 143.

Dans l'exemple décrit ici, ces moyens de positionnement sont des aimants portés par chaque diasporamètre 150, 152 et destinés à coopérer avec des éléments magnétiques (ici des aimants) du sous-système concerné 120, 122, comme expliqué plus loin en référence à la figure 12.

Chaque diasporamètre 150, 152 comporte deux montures guidées en rotation, dentées à leur périphérie et portant dans un logement un prisme, les prismes des deux montures étant alignés. Chaque monture peut être entraînée en rotation par un actionneur dédié, composé par exemple d'un moteur (pas à pas ou à courant continu) et d'une vis sans fin.

Chaque diasporamètre 150, 152 peut en outre comporter un accéléromètre afin de pouvoir déterminer sa propre position sur la face avant 142 de l'enceinte du sous-système concerné 120, 122.

Comme visible en figure 8, le support orientable 104 présente, par exemple dans la région centrale de la traverse 116, un moyen d'éclairage 154 (par exemple réalisé au moyen d'un émetteur/récepteur infrarouge), utilisable en particulier lors de la mesure d'acuité en vision de près, et un dispositif émetteur/récepteur 156 associé à une carte électronique de commande 160 comme expliqué ci-après en référence à la figure 10.

La figure 10 présente de manière détaillée le support orientable 104 sous forme d'un écorché.

La traverse 116 loge la carte électronique de commande 160, disposée ici horizontalement et sur la quasi-totalité de la longueur de la traverse 116. La carte électronique de commande 160 porte notamment le moyen d'éclairage 154 et le dispositif émetteur/récepteur 156, ici au contact d'une face avant de la traverse 116 munie d'orifices respectivement au droit du moyen d'éclairage 154 et du dispositif émetteur/récepteur 156.

La traverse 116 loge également, respectivement de part et d'autre de la carte électronique de commande 160 (dans le sens de la longueur de la traverse 116), un premier moteur 161 et un second moteur 162 : le premier moteur 161 permet d'entraîner le déplacement du premier bras articulé 124 autour de son axe de rotation 164 (orienté comme déjà indiqué selon la direction M d'extension du montant 114); le second moteur 162 permet d'entraîner le déplacement du second bras articulé 126 autour de son axe de rotation 166 (parallèle à l'axe 164 de rotation du premier bras articulé et à la direction M).

Chacun des premier et second moteurs 161, 162 est monté à une extrémité de la carte électronique de commande 160 et est commandé par celle-ci en fonction des instructions reçues à travers le dispositif émetteur/récepteur 156.

Comme bien visible sur la figure 10, le déplacement des bras articulés 124, 126 du fait de l'actionnement des moteurs 161, 162 permet de faire converger les axes optiques des sous-systèmes 120, 122 (portés respectivement par les bras articulés 124, 126 comme déjà indiqué), notamment de manière adaptée à la vision de près. On comprend que les bras articulés 124, 126 peuvent de manière générale être déplacés vers l'avant ou vers l'arrière du plan général du réfracteur.

L'angle de convergence Φ à utiliser pour une mesure d'acuité visuelle à une distance D (distance entre la pupille et l'échelle d'acuité) et pour une personne ayant un demi-écart pupillaire dPD pour l'œil concerné est donné par la formule : Φ = arctan(dPD/D).

Pour pouvoir s'adapter aux plus grands écarts pupillaires généralement rencontrés (80 mm) et à un objet observé (échelle d'acuité) à une distance D = 350 mm, on prévoit par exemple que chaque sous-système 120, 122 puisse avoir un mouvement de rotation autour de l'axe associé 164, 166 allant jusqu'à une valeur limite comprise entre 5° et 20°, ici 6,5°.

Le support orientable 104 porte également (ici à l'intérieur du montant 112) le moteur 170 qui permet, comme indiqué plus haut, de régler l'inclinaison du support orientable 104 par rapport à la partie fixe 102.

Le moteur 170 est également relié à la carte électronique de commande 160 et peut ainsi être piloté par des instructions reçues à travers le dispositif émetteur/récepteur 156. Un système de codage (roue codée/capteur), également lié à la carte électronique de commande 160, est éventuellement placé dans la partie inférieure du montant 112 afin de connaître en permanence l'inclinaison du support orientable 104 par rapport à la partie fixe 102.

La carte électronique 160 et les moteurs 161, 162, 170 sont par exemple alimentés au moyen d'un circuit d'alimentation connecté sur le réseau électrique du praticien du côté de la partie fixe 102 et qui rejoint le support orientable 104 dans la zone de montage en rotation du support orientable 104 sur la partie fixe 102.

Chaque montant 112, 114 du support orientable 104 porte également un dispositif de capture d'image 168, 169, ici une caméra (utilisant par exemple un capteur CMOS muni d'un objectif de type infrarouge ou visible) reliée à la carte électronique de commande 160 par une liaison filaire, monté de sorte que son axe de prise de vue soit substantiellement parallèle à l'axe de rotation H du support orientable 104 par rapport à la partie fixe 102 : chaque dispositif de capture d'image 168, 169 a ainsi dans son champ un profil du visage au niveau de l'œil concerné et la face arrière 144 de l'enceinte du sous-système 120, 122 concerné au niveau de l'oculaire 121, 123 concerné.

Ainsi, pour l'œil gauche et pour l'œil droit, la carte électronique de commande 160 peut traiter l'image prise par le dispositif de capture d'image 168, 169 concerné : d'une part, cette image peut être émise à travers le dispositif émetteur/récepteur 156 à un système extérieur, tel qu'un ordinateur du praticien, afin par exemple que le praticien puisse vérifier que les yeux du patient sont bien positionnés au centre des oculaires 121, 123 ; d'autre part, la carte électronique de commande 160 peut mesurer, sur la base de cette image, la distance entre le sommet de la cornée de l'œil concerné et la face arrière 144 de l'enceinte du sous-système 120, 122 concerné afin de corriger éventuellement les consignes en puissance du dispositif de compensation visuelle concerné pour tenir compte de cette distance.

En effet, en prenant l'exemple d'une puissance sphérique S de focale équivalente F, une erreur de positionnement ε revient à avoir une correction de focale F', équivalente à une puissance sphérique S', avec :

$$F' = F - \varepsilon \quad \text{et} \quad S' = S\left( \frac{1}{1 - \dfrac{\varepsilon}{F}} \right),$$

ce qui donne en première approximation $S' = S \cdot (1 + \varepsilon \cdot S)$.

La carte électronique de commande 160 enverra donc, à chacun des dispositifs de compensation visuelle, des consignes de puissance sphérique et cylindrique qui dépendront non seulement des puissances sphériques et cylindriques recherchées par le praticien, mais également de la distance œil - dispositif (ici cornée - face arrière 144) mesurée par le dispositif de capture d'image 168, 169.

La figure 11 représente de manière détaillée le montage du sous-système 122 destiné à la mesure de l'acuité visuelle de l'œil gauche du patient sur le bras articulé 126.

Le bras articulé 126 porte à son extrémité externe (c'est-à-dire, pour le sous-système 122 destiné à l'œil gauche, son extrémité gauche vu côté patient comme sur la figure 11) un moteur 180 (ici un moteur pas-à-pas) conçu pour entraîner en rotation une tige filetée 182 sur laquelle est monté un coulisseau 184 pourvu d'un filetage interne complémentaire de celui de la tige filetée 182.

Le coulisseau 184 se prolonge vers le bas par une extension 185 qui s'étend à travers une gorge formée dans la paroi inférieure du bras articulé 126 et à l'extrémité inférieure de laquelle est monté le sous-système 122.

Comme cela sera précisé plus loin en référence à la figure 12, le sous-système 122 comprend un dispositif de compensation visuelle 200 tel que celui décrit plus haut en référence aux figures 1 à 5, orienté de sorte que son espace parallélépipédique de réception des moteurs se situe latéralement et à l'extérieur (ici à gauche vu côté patient) de son espace cylindrique de réception des éléments optiques, c'est-à-dire de l'oculaire 123 (qui correspond à la fenêtre optique 145 formée dans la face arrière 144 de l'enceinte du sous-système 122, face arrière 144 formée en cet endroit par le boîtier 12 du dispositif de compensation visuelle 200).

Autrement dit, le dispositif de compensation visuelle 200 est positionné au sein du sous-système 122 de sorte que l'axe Z visible sur les figures 2 à 4 soit parallèle à l'axe de rotation horizontal H (l'axe Y des figures 2 à 4 étant quant à lui parallèle à la direction M d'extension du montant 112).

Le sous-système 122 est précisément monté sur l'extension 185 du coulisseau 184 au niveau du dispositif de compensation visuelle 200, dans la partie de jonction entre l'espace parallélépipédique de réception des moteurs et l'espace cylindrique de réception des éléments optiques.

Le moteur 180 est relié à la carte électronique de commande 160 et peut ainsi être commandé sur la base d'instructions reçues à travers le dispositif émetteur/récepteur 156. Par ailleurs, un système de codage 186 (roue codée/capteur), également relié à la carte électronique de commande 160 permet de connaître précisément la position angulaire de la tige filetée 182 et donc la position du coulisseau 184 et du sous-système 122.

L'activation du moteur 180 permet la mise en rotation de la tige filetée 182 et par conséquent le déplacement du coulisseau 184 et du sous-système 122 le long du bras articulé 126.

Ainsi, le sous-ensemble 122 peut notamment prendre deux positions : une position réglable adaptée à la morphologie du patient (telle que l'œil du patient et l'oculaire 123 sont alignés) et une position escamotée, dans laquelle le coulisseau 184 est placé à sa position d'extrémité extérieure (à gauche sur la figure 11) et le sous-système 122 est donc en dehors du champ de vision du patient.

La position escamotée peut être utilisée par exemple pour mesurer l'acuité visuelle du patient sans correction ou pour positionner le réfracteur à une hauteur adaptée pour le patient (puisque le praticien verra alors sans difficulté les yeux du patient), le sous-ensemble 122 pouvant alors être amené en position de travail dans un second temps.

Comme déjà indiqué, le montage du sous-système 120 sur le bras articulé 124 et son déplacement sont réalisés de manière analogue à ce qui vient d'être décrit pour le sous-système 122 sur le bras articulé 126.

La figure 12 représente le sous-système 122 conçu pour la mesure d'acuité visuelle de l'œil gauche du patient.

Comme indiqué ci-dessus, ce sous-système 122 comprend notamment un dispositif de compensation visuelle 200 tel que celui décrit plus haut en référence aux figures 1 à 5.

Le sous-système 122 est par ailleurs clos par une enceinte dont la face arrière 144 est formée par une partie du boîtier 12 du dispositif de compensation visuelle 200.

Entre le dispositif de compensation visuelle 200 et la face avant 142 de l'enceinte (dans la direction de l'axe optique), le sous-système 122 porte une roue interne 190 et une roue externe 192 centrées sur un axe de rotation commun parallèle à l'axe optique et distinct de celui-ci (l'axe optique étant celui du dispositif de compensation visuelle 200, situé au centre de l'oculaire 123).

La roue interne 190 porte par exemple une pluralité d'éléments optiques (par exemple partie d'occultation, filtre polariseur, filtre coloré, filtre de Maddox, prisme de découplage) dont l'un peut sélectivement être placé devant l'axe optique. La roue interne 190 présente également un passage libre qui peut lui aussi être positionné devant l'axe optique afin que le traitement optique apporté par le sous-système 122 corresponde à celui généré par le dispositif de compensation visuelle 200.

Dans le mode de réalisation décrit ici où le sous-système 122 porte sur sa face avant 142 un diasporamètre 152 déplaçable sur la face avant 142, la roue externe 192 porte par exemple des éléments magnétiques (ici des aimants) qui coopèrent comme déjà indiqué avec des aimants du diasporamètre de sorte qu'un déplacement de la roue externe 192 autour de l'axe commun précité entraîne le mouvement du diasporamètre 152 entre sa première position (où les prismes sont situés sur l'axe optique) et sa seconde position (où le diasporamètre laisse libre le champ de vision défini par l'oculaire 123).

La roue interne 190 et/ou la roue externe 192 sont par exemple montées dans un boîtier amovible muni d'une lame de protection anti-poussière. Le praticien peut ainsi changer les éléments optiques (filtre, prisme, partie d'occultation) présents dans le sous-système 122.

La face avant 142 présente un décrochement 195 afin de réduire le volume du sous-système 122 dans sa partie inférieure afin de limiter la longueur du trajet optique dans le produit au bénéfice de la largeur du champ de vision.

La roue interne 190 et la roue externe 192 sont en effet ici munies de dents sur leur périphérie, sur lesquelles vient engrener un pignon entraîné par le moteur d'entraînement correspondant 194, 196.

Les moteurs d'entraînement 194, 196 sont commandés par la carte électronique de commande 160. Un système de codage absolu non représenté permet de réaliser l'initialisation de la roue interne 190 et de la roue externe 192 de façon dissociée.

Grâce à la conception adoptée, le sous-système 122 est de dimensions réduites, typiquement inférieures à 120 mm en hauteur (c'est-à-dire selon la direction M) et en largeur (c'est-à-dire selon la direction H) et inférieure à 60 mm selon l'épaisseur (c'est-à-dire selon l'axe optique X du dispositif de compensation visuelle 200) ; dans l'exemple décrit ici, chaque sous-système est de dimensions 80 mm en hauteur, 80 mm en largeur et 43 mm en épaisseur.

On décrit à présent un exemple d'utilisation du réfracteur qui vient d'être décrit.

Les sous-systèmes 120, 122 sont tout d'abord positionnés en position escamotée (c'est-à-dire à leur position horizontale la plus distante d'un plan vertical médian passant par le milieu de la traverse 116). Le support orientable 104 est quant à lui en position verticale (c'est-à-dire dans la configuration du réfracteur adaptée à la vision de loin comme représenté en figure 6).

Le patient place alors sa tête devant le réfracteur.

Le praticien peut ainsi régler la position en hauteur du réfracteur par des moyens conventionnels non discutés ici, ceci d'autant plus facilement qu'il voit les yeux du patient du fait que les sous-systèmes 120, 122 sont en position escamotée.

Le patient place alors son front sur les moyens de positionnement 130 et le praticien commande (par exemple à l'aide d'un ordinateur qui envoie des instructions adaptées à la carte électronique de commande 160) l'activation des moteurs 180 de manière à déplacer les sous-systèmes 120, 122 horizontalement le long des bras articulés 124, 126 jusqu'à ce que les oculaires 121, 123 soient situés en face des yeux du patient.

Le réfracteur est à ce moment configuré pour mesurer l'acuité visuelle du patient en vision de loin.

Le praticien peut alors commander (également au moyen de l'ordinateur qui émet des instructions à destination de la carte électronique de commande 160) le positionnement des différents éléments de chaque sous-système 120, 122, et notamment :

- en fonction des consignes de corrections sphérique et cylindrique pour un œil donné reçues par la carte électronique de commande 160, la carte électronique de commande 160 émet des consignes au dispositif de compensation visuelle associé à l'œil concerné, en tenant compte comme indiqué ci-dessus de la distance entre l'œil et le dispositif de compensation visuelle mesurée grâce au dispositif de capture d'image ;
- la carte électronique de commande 160 commande éventuellement la rotation du moteur 194 afin de placer le cas échéant un filtre ou un prisme supplémentaire selon l'axe optique ;
- la carte électronique de commande 160 commande éventuellement la rotation du moteur 196 afin de placer si nécessaire les prismes du diasporamètre 150, 152 devant l'oculaire 121, 123 concerné, ainsi que la rotation des moteurs du diasporamètre 150, 152 afin d'obtenir la correction prismatique requise.

Lorsqu'une correction adaptée à l'amétropie du patient en vision de loin est trouvée, le praticien commande par exemple la mémorisation des valeurs de correction (sphérique, cylindrique et prismatique) en association avec l'inclinaison courante du support orientable (déterminée par exemple par la carte électronique de commande 160 grâce au système de codage associé au moteur 170 ou par les dispositifs de compensation visuelle 200 grâce à l'information d'orientation délivrée par l'accéléromètre), ici une inclinaison nulle.

Le praticien commande alors l'inclinaison du support orientable 104 (au moyen de sa rotation par rapport à la partie fixe 102 autour de l'axe horizontal H, réalisée au moyen du moteur 170 commandé par la carte électronique de commande 160 en fonction d'instructions reçues via le dispositif émetteur/récepteur 156 de l'ordinateur du praticien).

Le réfracteur est alors dans sa configuration dans laquelle le support orientable 104 est incliné, par exemple de 30° par rapport à la verticale, afin de mesurer l'acuité visuelle du patient en vision de près. On comprend que l'on peut également positionner le support orientable 104 dans d'autres positions intermédiaires afin de mesure l'acuité visuelle en vue intermédiaire.

Comme déjà indiqué, du fait que l'axe horizontal H est placé au niveau du centre de rotation de l'œil, les oculaires 121, 123 suivent le regard du patient et le réfracteur est donc prêt pour mesurer l'acuité visuelle du patient en vision de près sans que celui-ci ait à se repositionner.

Le praticien peut alors commander (comme déjà expliqué ci-dessus pour la vision de loin) le positionnement des différents éléments de chaque sous-système 120, 122.

Lorsqu'une correction adaptée à l'amétropie du patient en vision de près est trouvée, le praticien commande par exemple la mémorisation des valeurs de correction (sphérique, cylindrique et prismatique) en association avec l'inclinaison courante du support orientable (déterminée par exemple par la carte électronique de commande 160 grâce au système de codage associé au moteur 170 ou par les dispositifs de compensation visuelle 200 grâce à l'information d'orientation délivrée par l'accéléromètre), ici une inclinaison de 30°.

Les valeurs de correction mémorisées (tant pour la vision de loin que pour la vision de près, et éventuellement pour la vision intermédiaire) pourront alors être utilisées pour la réalisation de lentilles ophtalmiques adaptées à la vue du patient.

**Revendications**

1. Réfracteur (100) comprenant :

   - une enceinte comprenant une face avant (142) présentant une première fenêtre optique (143) et une face arrière (144) présentant une seconde fenêtre optique (145) alignée avec la première fenêtre optique (143) selon un axe optique d'observation ;
   - au moins un dispositif de compensation visuelle (10 ; 200) permettant d'observer selon l'axe optique d'observation,
   - le dispositif de compensation visuelle (10 ; 200) comprend, entre la première fenêtre et la seconde fenêtre, un premier élément optique (6) de puissance sphérique selon l'axe optique variable, et en ce que
   - l'enceinte est montée sur un support orientable (104) mobile en rotation par rapport à une partie fixe (102) autour d'un axe horizontal (H),

   **caractérisé en ce que**
   le réfracteur comprend au moins un dispositif de capture d'image (168 ; 169) ayant un axe de prise de vue, dans lequel le dispositif de capture d'image (168 ; 169) est monté dans le réfracteur (10) de sorte que l'axe de prise de vue soit essentiellement parallèle audit axe horizontal (H) ce qui permet de surveiller en permanence la situation de l'œil par rapport au dispositif de compensation visuelle.

2. Réfracteur selon la revendication 1, dans lequel le dispositif de compensation visuelle (10 ; 200) est conçu pour générer une correction cylindrique d'axe de cylindre variable et de puissance variable.

3. Réfracteur selon la revendication 1 ou 2, dans lequel le dispositif de compensation visuelle (10 ; 200) comprend un second élément optique (2) de puissance cylindrique et un troisième élément optique (4) de puissance cylindrique, le second élément optique (2) et le troisième élément optique (4) étant réglables en rotation autour de l'axe optique indépendamment l'un de l'autre.

4. Réfracteur selon l'une des revendications 1 à 3, dans lequel l'enceinte est montée sur un bras articulé (124 ; 126) par rapport au support orientable (104) autour d'un axe perpendiculaire (M) à l'axe horizontal (H).

5. Réfracteur selon l'une des revendications 1 à 4, dans lequel l'enceinte est montée sur le support orientable (104) à une position réglable selon l'axe horizontal (H).

6. Réfracteur selon l'une des revendications 1 à 5, dans lequel une roue (190 ; 192) est montée mobile en rotation dans l'enceinte autour d'un axe parallèle à l'axe optique et porte au moins un élément complémentaire destiné à être placé devant l'axe optique.

7. Réfracteur selon la revendication 6, dans lequel l'élément complémentaire est un filtre ou un prisme ou une ouverture ou un cache.

8. Réfracteur selon l'une des revendications 1 à 7, comprenant des moyens de montage mobile d'un diasporamètre (150 ; 152) sur la face avant (142) de l'enceinte dans une première position, dans laquelle au moins un prisme du diasporamètre (150 ; 152) est aligné avec l'axe optique, et dans une seconde position, dans laquelle le diasporamètre (150 ; 152) laisse libre la première fenêtre optique.

9. Réfracteur selon l'une des revendications 1 à 8, dans lequel l'enceinte présente une face inférieure (146) et dans lequel la distance, en projection dans un plan vertical, entre l'axe optique et la face inférieure (146) est inférieure à 30 mm.

10. Réfracteur selon l'une des revendications 1 à 9, dans lequel la position du support orientable (104) est réglable en rotation autour de l'axe horizontal (H) au moyen d'un actionneur (170).

**11.** Réfracteur selon l'une des revendications 1 à 10, dans lequel la partie fixe (102) comprend des moyens de positionnement (130) d'une partie de la tête d'un utilisateur.

**12.** Réfracteur selon la revendication 11, dans lequel la partie fixe (102) est dimensionnée de sorte que ledit axe horizontal (H) passe par les yeux dudit utilisateur.

**13.** Réfracteur selon la revendication 12, dans lequel la partie fixe est dimensionnée de sorte que l'axe horizontal (H) passe par le centre de rotation d'au moins un œil dudit utilisateur.

**14.** Réfracteur selon l'une des revendications 1 à 13, dans lequel le dispositif de capture d'image (168 ; 169) est conçu pour mesurer la distance entre l'œil et le dispositif de compensation visuelle (200).

**15.** Réfracteur selon la revendication 14, dans lequel le dispositif de compensation visuelle est conçu pour commander la puissance sphérique du premier élément optique (6) en fonction de la distance mesurée.

**16.** Procédé de mesure de la réfraction au moyen d'un réfracteur selon l'une des revendications 1 à 15, comprenant les étapes suivantes :

- inclinaison du support orientable (104) par rapport à la partie fixe (102) ;
- réglage de la puissance sphérique du premier élément optique (6) ;
- mémorisation de la puissance sphérique réglée.

**17.** Procédé de mesure selon la revendication 16, dans lequel le réglage de la puissance sphérique est effectué en fonction d'une distance entre un œil d'un utilisateur et le dispositif de compensation visuelle mesurée par un dispositif de capture d'image (168 ; 169).

**Patentansprüche**

**1.** Phoropter (100), der enthält:

- ein Gehäuse, das eine Vorderseite (142), die ein erstes optisches Fenster (143) aufweist, und eine Rückseite (144) enthält, die ein zweites optisches Fenster (145) aufweist, das gemäß einer optischen Beobachtungsachse mit dem ersten optischen Fenster (143) ausgerichtet ist;
- mindestens eine Vorrichtung zur visuellen Kompensation (10; 200), die es ermöglicht, gemäß der optischen Beobachtungsachse zu beobachten,
- wobei die Vorrichtung zur visuellen Kompensation (10; 200) zwischen dem ersten Fenster und dem zweiten Fenster ein erstes optisches Element (6) mit sphärischer Brechkraft gemäß der variablen optischen Achse enthält, und dass
- das Gehäuse auf einen ausrichtbaren Träger (104) montiert ist, der bezüglich eines ortsfesten Teils (102) um eine waagrechte Achse (H) drehbeweglich ist,

**dadurch gekennzeichnet, dass**
der Phoropter mindestens eine Bilderfassungsvorrichtung (168; 169) enthält, die eine Bildaufnahmeachse hat, wobei die Bilderfassungsvorrichtung (168; 169) so in den Phoropter (10) montiert ist, dass die Bildaufnahmeachse im Wesentlichen parallel zur waagrechten Achse (H) ist, was es ermöglicht, die Situation des Auges bezüglich der visuellen Kompensationsvorrichtung durchgehend zu überwachen.

**2.** Phoropter nach Anspruch 1, wobei die visuelle Kompensationsvorrichtung (10; 200) konzipiert ist, um eine Zylinderkorrektur mit variabler Zylinderachse und variabler Brechkraft zu erzeugen.

**3.** Phoropter nach Anspruch 1 oder 2, wobei die visuelle Kompensationsvorrichtung (10; 200) ein zweites optisches Element (2) mit zylindrischer Brechkraft und ein drittes optisches Element (4) mit zylindrischer Brechkraft enthält, wobei das zweite optische Element (2) und das dritte optische Element (4) unabhängig voneinander in Drehung um die optische Achse einstellbar sind.

4. Phoropter nach einem der Ansprüche 1 bis 3, wobei das Gehäuse auf einen bezüglich des ausrichtbaren Trägers (104) um eine Achse (M) lotrecht zur waagrechten Achse (H) angelenkten Arm (124; 126) montiert ist.

5. Phoropter nach einem der Ansprüche 1 bis 4, wobei das Gehäuse in einer gemäß der waagrechten Achse (H) einstellbaren Stellung auf den ausrichtbaren Träger (104) montiert ist.

6. Phoropter nach einem der Ansprüche 1 bis 5, wobei ein Rad (190; 192) im Gehäuse um eine Achse parallel zur optischen Achse drehbeweglich montiert ist und mindestens ein komplementäres Element trägt, das dazu bestimmt ist, vor der optischen Achse angeordnet zu werden.

7. Phoropter nach Anspruch 6, wobei das komplementäre Element ein Filter oder ein Prisma oder eine Öffnung oder eine Abdeckung ist.

8. Phoropter nach einem der Ansprüche 1 bis 7, der Einrichtungen zur beweglichen Montage eines Diasporameters (150; 152) an der Vorderseite (142) des Gehäuses in einer ersten Stellung, in der mindestens ein Prisma des Diasporameters (150; 152) mit der optischen Achse ausgerichtet ist, und in einer zweiten Stellung enthält, in der das Diasporameter (150; 152) das erste optische Fenster frei lässt.

9. Phoropter nach einem der Ansprüche 1 bis 8, wobei das Gehäuse eine Unterseite (146) aufweist, und wobei der Abstand, in Projektion in einer senkrechten Ebene, zwischen der optischen Achse und der Unterseite (146) geringer als 30 mm ist.

10. Phoropter nach einem der Ansprüche 1 bis 9, wobei die Stellung des ausrichtbaren Trägers (104) mittels eines Stellglieds (170) in Drehung um die waagrechte Achse (H) einstellbar ist.

11. Phoropter nach einem der Ansprüche 1 bis 10, wobei der ortsfeste Teil (102) Positioniereinrichtungen (130) eines Teils des Kopfes eines Benutzers enthält.

12. Phoropter nach Anspruch 11, wobei der ortsfeste Teil (102) so bemessen ist, dass die waagrechte Achse (H) durch die Augen des Benutzers verläuft.

13. Phoropter nach Anspruch 12, wobei der ortsfeste Teil so bemessen ist, dass die waagrechte Achse (H) durch das Drehzentrum mindestens eines Auges des Benutzers verläuft.

14. Phoropter nach einem der Ansprüche 1 bis 13, wobei die Bilderfassungsvorrichtung (168; 169) konzipiert ist, um den Abstand zwischen dem Auge und der visuellen Kompensationsvorrichtung (200) zu messen.

15. Phoropter nach Anspruch 14, wobei die visuelle Kompensationsvorrichtung konzipiert ist, um die sphärische Brechkraft des ersten optischen Elements (6) abhängig vom gemessenen Abstand zu steuern.

16. Messverfahren der Refraktion mittels eines Phoropters nach einem der Ansprüche 1 bis 15, das die folgenden Schritte enthält:

   - Neigung des ausrichtbaren Trägers (104) bezüglich des ortsfesten Teils (102);
   - Einstellen der sphärischen Brechkraft des ersten optischen Elements (6);
   - Speichern der eingestellten sphärischen Brechkraft.

17. Messverfahren nach Anspruch 16, wobei die Einstellung der sphärischen Brechkraft abhängig von einem zwischen einem Auge eines Benutzers und der visuellen Kompensationsvorrichtung gemessenen Abstand von einer Bilderfassungsvorrichtung (168; 169) ausgeführt wird.

**Claims**

1. Refractor (100) comprising:

   - an enclosure comprising a front face (142) containing a first optical window (143) and a back face (144) containing a second optical window (145) aligned with the first optical window (143) along an optical axis of observation;
- at least one vision compensating device (10; 200) making it possible to observe along the optical axis of observation;
- the vision compensating device (10; 200) comprises, between the first window and the second window, a first optical element (6) having a spherical power along the variable optical axis; and in that
- the enclosure is mounted on an orientable holder (104) that is rotatable relative to a stationary portion (102) about a horizontal axis (H);

**characterized in that**

the refractor comprises at least one image capturing device (168; 169) having a line of sight, in which the image capturing device (168; 169) is mounted in the refractor (10) so that the line of sight is essentially parallel to said horizontal axis (H), this allowing the location of the eye relative to the vision compensating device to be monitored without interruption.

2. Refractor according to Claim 1, in which the vision compensating device (10; 200) is designed to generate a cylindrical correction of variable cylinder axis and of variable power.

3. Refractor according to Claim 1 or 2, in which the vision compensating device (10; 200) comprises a second optical element (2) of cylindrical power and a third optical element (4) of cylindrical power, the second optical element (2) and the third optical element (4) being rotatably adjustable about the optical axis independently of each other.

4. Refractor according to one of Claims 1 to 3, in which the enclosure is mounted on an arm (124; 126) hinged relative to the orientable holder (104) about an axis (M) perpendicular to the horizontal axis (H).

5. Refractor according to one of Claims 1 to 4, in which the enclosure is mounted on the orientable holder (104) in a position that is adjustable along the horizontal axis (H).

6. Refractor according to one of Claims 1 to 5, in which a wheel (190; 192) is rotatably mounted in the enclosure about an axis parallel to the optical axis and bears at least one complementary element intended to be placed in front of the optical axis.

7. Refractor according to Claim 6, in which the complementary element is a filter or a prism or an aperture or a shield.

8. Refractor according to one of Claims 1 to 7, comprising means for movably mounting a diasporameter (150; 152) on the front face (142) of the enclosure in a first position, in which position at least one prism of the diasporameter (150; 152) is aligned with the optical axis, and in a second position, in which position the diasporameter (150; 152) leaves free the first optical window.

9. Refractor according to one of Claims 1 to 8, in which the enclosure has a lower face (146) and in which the distance, in projection onto a vertical plane, between the optical axis and the lower face (146) is smaller than 30 mm.

10. Refractor according to one of Claims 1 to 9, in which the position of the orientable holder (104) is rotatably adjustable about the horizontal axis (H) by means of an actuator (170).

11. Refractor according to one of Claims 1 to 10, in which the stationary portion (102) comprises means (130) for positioning a portion of the head of a user.

12. Refractor according to Claim 11, in which the stationary portion (102) is dimensioned so that said horizontal axis (H) passes through the eyes of said user.

13. Refractor according to Claim 12, in which the stationary portion is dimensioned so that the horizontal axis (H) passes through the centre of rotation of at least one eye of said user.

14. Refractor according to one of Claims 1 to 13, in which the image capturing device (168; 169) is designed to measure the distance between the eye and the vision compensating device (200).

15. Refractor according to Claim 14, in which the vision compensating device is designed to control the spherical power of the first optical element (6) depending on the measured distance.

16. Method for measuring refraction by means of a refractor according to one of Claims 1 to 15, comprising the following steps:

- inclining the orientable holder (104) relative to the stationary portion (102);
- adjusting the spherical power of the first optical element (6); and
- storing the adjusted spherical power in memory.

17. Measuring method according to Claim 16, in which the spherical power is adjusted depending on a distance between an eye of a user and the vision compensating device, which distance is measured by an image capturing device (168; 169).

## Fig.1

2

6

$Y_1$  $Y_2$  X  $Y_0$

4

10

12  18  Z  16  14

50

36

58

6  4

2

X

24

22  52

26

54

27

## Fig.2

50

42  52

70

68

CEL1

REC
S,C,$\alpha$

CALC
$\alpha_1,\alpha_2,S_V$

CMD
MOT

$MOT_1$

60

66

54

CEL2

$MOT_2$

44

CEL3

TPT

INCLIN

46

$MOT_3$

56

## Fig.5

62  64

**Fig.3**

**Fig.4**

**Fig.6**

**Fig.7**

**Fig.8**

104  154  124  126  156  116
150  140  152  112
114  H
d
120  121  142  143  123  146  122

**Fig.9**

110  130
106  126  116  108
140  H
144  122  123  145  121  124  120
146

**Fig.10**

**Fig.11**

**Fig.12**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20040032568 A
- EP 2034338 A